# EUROPÄISCHE PATENTANMELDUNG

(11) **EP 4 410 946 A2**
(43) Veröffentlichungstag der Anmeldung: **07.08.2024**
(21) Anmeldenummer: 24155477.3
(22) Anmeldetag: 02.02.2024
(51) Int. Cl.: C12M 1/00, C05F 17/50

(54) **VERFAHREN ZUR STOFFLICHEN UND ENERGETISCHEN VERWERTUNG VON FERMENTATIONSRÜCKSTÄNDEN AUS DER METHANFERMENTATION VON ENERGIEPFLANZEN**

(30) Priorität: 02.02.2023 DE 102022003659
(71) Anmelder: LUCE Patent GmbH, 52499 Baesweiler (DE)
(72) Erfinder: Auerbach, Hans-Joachim, 07549 Gera (DE)
(74) Vertreter: Gottfried, Hans-Peter

(57) **Zusammenfassung**

Verfahren zur stofflichen und energetischen Verwertung von festen Rückständen der Methanfermentation von Energiepflanzen aus Fermentationsrückständen und/oder tierischen Exkrementen und/oder Pflanzenresten mittels einer für die Nassfermentation geeigneten Biosuspension mit einer hydrolytischen Vorbehandlung und mehrstufiger anaerober Behandlung. Nach der Erfindung werden die festen Rückstände aus der Methanfermentation von Energiepflanzen mit einem Trockenmassegehalt von wenigstens 25 % zugeführt, die zugeführte Trockenmasse beträgt zwischen 40 und 100 % der Trockenmasse, die hydrolytische Vorbehandlung wird mittels Intensivrotte der Einsatzstoffe vor dem Suspensionsprozess und/oder durch Sauerstoffbelastung werden der Einsatzstoffe vorgenommen, bei der Herstellung einer Biosuspension Filtrate und/oder Kondensate aus der Aufbereitung der Rückstände der Methanfermentation des Gemischs als Suspendierungsmittel eingesetzt, die aerobe hydrolytische Vorbehandlung der Biosuspension wird von der Fermentation getrennt unter Zusatz von 0,6 bis 1,4 m³ Sauerstoff je kg organischer Trockensubstanz vorgenommen und bei Nutzung der Intensivrotte wird für die hydrolytische Vorbehandlung der Einsatzstoffe ohne Sauerstofflimitierung die Behandlungszeit zwischen 0,5 und 48 Stunden gewählt.

## Beschreibung

### GEBIET DER TECHNIK

Die Erfindung betrifft ein Verfahren zur stofflichen und energetischen Verwertung von Fermentationsrückständen aus der Methanfermentation von Energiepflanzen, wobei in einem ersten Schritt zumindest aus den Fermentationsrückständen in einem Suspensionsprozess unter Zugabe von Prozessflüssigkeit eine für die Nassfermentation geeignete Biosuspension hergestellt wird, in einem zweiten Schritt eine hydrolytische Vorbehandlung und in einem dritten Schritt eine anaerobe Methanbildung in von der hydrolytischen Vorbehandlung räumlich getrennten Prozessbehältern, in denen der jeweilige Prozess abläuft, erfolgen, wobei die Prozessbehälter kulturerhaltend ausgestattet oder kulturerhaltend betrieben werden, sodass die Mikrokultur in Qualität und Quantität stabil bleibt, wobei zumindest ein energetisch verwertbares methanhaltiges Biogas und ein stofflich verwertbares Düngemittel gebildet werden.

Dabei ist unter Methanfermentation zu verstehen, dass organische Materialien in einer Abfolge von Prozessschritten, zumindest Hydrolyse und anaerobe Methanbildung, die organischen Materialien, die auch als Einsatzstoffe (oder als Gärsubstrat, wenn bereits im Prozess) bezeichnet werden, zum Teil abgebaut werden. Aus der Methanfermentation resultiert und methanhaltiges Gas, Biogas genannt. Die Fermentationsrückstände im Sinne der Erfindung sind demnach die nicht abgebauten Teile der zuvor der Methanfermentation zugeführten Energiepflanzen. Das Biogas kann nach Aufbereitung in ein Gasnetz eingespeist werden oder unmittelbar verbrannt werden, was üblicherweise in einem Motor mit Kraft-Wärme-Kopplung und gekoppeltem Generator, zusammen zumeist als Blockheizkraftwerk (kurz: BHKW) bezeichnet.

Energiepflanzen sind nachwachsende Rohstoffe, speziell für die energetische Nutzung angebaute Pflanzen. Für die energetische Nutzung mittels Methanfermentation kommt zum großen Teil Maissilage als Energiepflanzen zum Einsatz. Im Sinne der Erfindung wird jedoch sämtliche Biomasse, die im wesentlichen Pflanzen und Pflanzenteile umfasst, als Energiepflanzen betrachtet. Charakteristisch für Energiepflanzen ist ein hoher Anteil an lignocellulosen Fasern, die nach der Methanfermentation im Gärsubstrat vorliegen.

Dabei ist weiterhin unter Nassfermentation (auch Nassgärung genannt) der Einsatz eines Gärsubstrats mit einem Gehalt an Trockensubstanz (kurz: TS-Gehalt) von bis zu etwa 15 %, in Abgrenzung zur Trockenfermentation mit höheren, darüber liegenden TS-Gehalten, zu verstehen. Diese Grenze bedingt insbesondere eine andere Anlagentechnik, vor allem für das Umwälzen und Fördern des Gärsubstrats. Es wird weiterhin der Gehalt an organischer Trockensubstanz (oTS) betrachtet.

Als Biosuspension wird daher das Gemisch bezeichnet, das auf einen TS-Gehalt gebracht wurde, das eine Nassfermentation ermöglicht. Auf diesen Weise können auch Einsatzstoffe mit einem an sich zu hohen TS-Gehalt dennoch in einer Anlage verarbeitet werden, in der eine Nassfermentation abläuft.

Als Intensivrotte wird im Zusammenhang mit der vorliegenden Erläuterung der Erfindung ein aerober, unter Sauerstoffzutritt ablaufender intensiver Fäulnisprozess bezeichnet, insbesondere der intensive Sauerstoffkontakt und folgerichtig auch der aeroben Mikroorganismen mit den ununterbrochen bewegten Rohfaseroberflächen. Der intensive Charakter wird erreicht durch eine aktive Belüftung, vor allem aber eine erhöhte Temperatur, beispielsweise 70 °C.

Unter kulturerhaltend ist zu verstehen, dass die vorhandene und adaptierte Bakterienkultur an die herrschenden Bedingungen in den Prozessbehältern, wie dem Methanfermenter oder in anderen Bioreaktoren wie dem Hydrolysebehälter oder der Entschwefelung, angepasst sind und diese Bakterienkultur erhalten bleibt. Die Bakterienkultur ist eine Zusammensetzung einer großen Vielzahl von Bakterienarten und wird nachfolgend als Mikrokultur bezeichnet. Insbesondere muss bei der Kulturerhaltung verhindert werden, dass die Mikrokultur, die sich in den Einsatzstoffen befindet und von dieser nicht trennbar ist, nicht in so großer Menge ausgetragen wird, dass die Bakterienkultur zu stark ausgedünnt wird. Die Regeneration der Mikrokultur muss daher immer schneller sein als die Austauschrate des Inhalts des Prozessbehälters, ob dieser nun kontinuierlich oder diskontinuierlich betrieben wird.

Ein kulturerhaltend ausgestatteter Prozessbehälter mit hoher Austauschrate weist beispielsweise Aufwuchsflächen auf, insbesondere wenn der Volumenstrom der Stoffe hoch ist oder diese keine Fasern oder ähnliche Feststoffteile aufweisen, auf denen die Bakterienkultur halt finden kann. Dies ist beim Methanfermenter in der Regel nicht der Fall, da die Einsatzstoffe eher faserreich sind, vor allem auch beim Einsatz von nachwachsenden Rohstoffen oder deren Rückständen. Dann steht der kulturerhaltende Betrieb in Vordergrund, vor allem die Abstimmung zwischen der Regenerationszeit der Bakterienkultur und dem Volumenstrom. Insbesondere darf der Volumenstrom nicht so hoch sein, dass der gesamte Inhalt im Mittel schneller ausgetauscht ist, als sich die Bakterienkultur vollständig regeneriert. Die Regenation der Bakterienkultur hängt ihrerseits von den Bedingungen im Prozessbehälter ab, beispielsweise der Temperatur und dem Nahrungsangebot.

Ein mesophiles Milieu bezeichnet einen Temperaturbereich, der etwa bei 25 - 30 °C beginnt und bis 35 °C, teils bis 42 °C, reicht, während ein thermophiles Milieu einen Temperaturbereich etwa zwischen 50 °C und 57 °C bezeichnet.

Bei der Methanfermentation von Pflanzenteilen, insbesondere Energiepflanzen bleibt das enthaltene Potential an mineralischen Pflanzennährstoffen vollständig erhalten. Eine derartige technische Lösung wird für die Gewinnung von Bioenergie benötigt, die weder organische Lebens- oder Futtermittel, noch Ackerflächen für den zusätzlichen Anbau von Energiepflanzen voraussetzt.

### STAND DER TECHNIK

Die Gewinnung von methanhaltigen Brenngasen aus biogenem Material gilt als unverzichtbare Schlüsseltechnologie im Bereich der so genannten Energiegewinnung aus regenerativen Quellen. Sie ist als so genanntes Multitalent den anderen Techniken zur Nutzung regenerativer Energiequellen insbesondere dadurch überlegen, dass sie
- sowohl für die Versorgung mit elektrischer und thermischer Energie als auch für die Kraftstoffversorgung nutzbar ist,
- mit erprobten und verfügbaren technischen Mitteln speicherbar ist, wahlweise der energetischen Grundlast- und auch der Spitzenlastversorgung dienen kann,
- mit der Gewinnung besonders pflanzenverfügbarer organischer Stickstoff-Phosphor-Kalium-Schwefel-Düngemittel verbunden werden kann,
- zur Minderung der Emission von Klimaschadgasen beiträgt und
- über zumindest in urbanisierten und/oder industrialisierten Regionen bereits verfügbare und ausreichend leistungsfähige Energie-Verteilnetze dezentral versorgungswirksam eingesetzt werden kann.

Die Rückstände aus den in beachtlichen Stückzahlen aktiven landwirtschaftlichen Biogasanlagen für die Verwertung von tierischen Exkrementen und/oder so genannten Energiepflanzen, wie Ganzpflanzensilage, insbesondere Maissilage, werden als Wirtschaftsdünger eingesetzt, die dem Wirtschaftskreislauf in großen Mengen für die Pflanzendüngung zur Verfügung stehen.

Diese Rückstände zeichnen sich durch eine Reihe einzigartiger Besonderheiten aus:
- sie stehen in der jeweiligen Standortregion ganzjährig mit überwiegend gleichartigen Eigenschaften zur Verfügung,
- sie sind das Ergebnis einer regelmäßig mehrfachen biotechnologischen Behandlung der ursprünglichen Einsatzstoffe, wie die Verdauung im Tiermagen, die anaerobe Silierung und die ein- oder mehrstufige konventionelle Methanfermentation,
- ihnen fehlt der überwiegende Teil der ursprünglich in den Exkrementen und/oder den Pflanzenteilen enthaltenen und den Mikrokulturen vergleichsweise leicht zugänglichen Inhaltsstoffe, wie Rohfett, Rohprotein und den einfachen Kohlenstoffhydraten Zucker oder Stärke.

Allein in Deutschland werden in den wenigstens 8.000 aktiven Biogasanlagen zur Verarbeitung von nachwachsenden Rohstoffen trockensubstanzhaltige (TS-haltige) Fermentationsrückstände produziert, aus denen nahezu problemlos jährlich wenigstens 8.000 Anlagen * 12.000 t/a * 30 % TS * 0,88 t oTS/t TS = 25,3 Mio. t organische Trockensubstanz (oTS) gewonnen werden können.

Obwohl es sich dabei bereits um Fermentationsrückstände aus einer vorangegangenen Methanfermentation mit einem Energiegewinn von im Mittel wenigstens etwa 8.000 Anlagen * 1,3 MW_{th.} * 8.200 h/a = 85,3 TWh/a handelt, sind von den 25,3 Mio. t oTS/a bis auf die Ligningehalte mehr als 95 % der weiteren Methanfermentation zugänglich, mithin wenigstens 25,3 Mio. t oTS/a * 0,95 = 24,0 Mio. t oTS/a.

Ohne die anorganische Trockensubstanz als Träger des enthaltenen Pflanzennährstoffpotentials zu dezimieren, steht bereits bei Ansatz minimaler Erträge einer anschließenden weiteren Methanfermentation in Höhe von 3,5 MWh_{th.}/t oTS damit eine bereits erschlossene stoffliche und ganzjährig stabil nutzbare Reserve für die Gewinnung von regenerativer Bioenergie in Höhe von 24,0 Mio. t oTS/a * 3,5 MWh_{th.}/t oTS = 88,0 TWh/a zur Verfügung.

Diese Situation ist den Aktiven der regenerativen Energiewirtschaft bestens bekannt. Es hat deshalb auch nicht an Versuchen gemangelt, technische Lösungen für die Erschließung dieser besonderen Bioenergiereserve aufzufinden.

Die zahlreich entwickelten Verfahren
- zur hydrothermalen Carbonisierung (HTC),
- zum Trocknen und Verbrennen,
- zum Trocknen und Pyrolysieren,
- zum Trocknen und Vergasen sowie
- zum katalytischen Umwandeln der Biomasse bei höheren Drücken und Temperaturen zu Brenn- und oder Kraftstoffen

tragen nicht zu einer nachhaltigen Lösung bei, weil diese Techniken immer mit einem Verlust von beachtlichen Anteilen der in den Fermentationsrückständen enthaltenen anorganischen Pflanzennährstoffe verbunden sind und weil ein erheblicher Teil der aus der Kohlenstoffumsetzung gewinnbare Energie für die Deckung des erforderlichen Prozessenergiebedarfs eingesetzt werden muss.

Darüber hinausgehende Entwicklungen sind insbesondere der Patentliteratur entnehmbar.

Die DE 19937876 C2 beschreibt bereits ein Verfahren zur biologischen Umsetzung von organischen Stoffen zu Methangas, das durch die räumliche Trennung von hydrolytischer Vorbehandlung der eingesetzten biogenen Stoffe und der Methanfermentation der dabei gewonnenen Hydrolysate gekennzeichnet ist. Unabhängig davon, dass auch über 20 Jahre nach der Offenlegung dieses Verfahrensvorschlages noch kein praktisches Beispiel für das Ausschleusen nicht mikrobiell abbaubarer Anteile eines Gemischs vor der Methanfermentation oder für das Anreichern von Teilen der verfügbaren Mikrokultur in den Fermentationsrückständen mittels Membrantechnik mit Rückführung in die Methanfermentation bekannt geworden ist, weist die Offenlegung auf die Sinnhaftigkeit der aeroben und/oder der anaeroben hydrolytischen Vorbehandlung der verfügbaren biogenen Stoffe durch Begasung mittels Luft oder Kohlenstoffdioxid hin. Der Einsatz von Sauerstoff aus der eingeblasenen Luft würde allerdings zwangsläufig zur Verminderung der Methanausbeute und zu Problemen bei der weiteren Gasnutzung führen.

Die EP 2 188 230 B2 macht ein Verfahren zur Herstellung von humus- und nährstoffreichen sowie wasserspeichernden Böden oder Bodensubstraten für nachhaltige Landnutzungs- und Siedlungssysteme bekannt. Danach werden höhere Drücke oder Prozesstemperaturen von mehr als 70 °C nicht benötigt, dagegen jedoch der Zusatz von pyrogenem Kohlenstoff und eine die Milchsäuregärung bewirkende Starterkultur. Hinweise zum vollständigen Erhalt der mit der Biomasse eingesetzten Pflanzennährstoffe, insbesondere der Stickstoffverbindungen, sind dieser Offenbarung nicht zu entnehmen. Das Erzielen eines nennenswerten Quantums an Nutzenergie aus der Behandlung des zuzusetzenden pyrogenen Kohlenstoffs und der kohlenstoffhaltigen Biomasse sind nicht das erklärte Ziel der beschriebenen Entwicklung.

Die DE 10 2008 028 788 B4 offenbart eine Vorrichtung und ein Verfahren zur Umsetzung von Biomasse in gasförmige Produkte. Mit der beschriebenen technischen Lösung sollen die energetischen und die besonders anspruchsvollen Prozessanforderungen einer Thermolyse für die Umwandlung von feuchter Biomasse in gasförmige Produkte durch die Verfahrensführung in einem Druckreaktor zur Aufnahme überkritischen Wassers und einer Salzschmelze reduziert werden. Das Niveau einer "sanften" biotechnologischen Problemlösung lässt sich mit den vorgeschlagenen Verbesserungen jedoch unübersehbar nicht erreichen.

Mit der DE 10 2009 027 274 A1 wird ein chemisch-physikalisches Verfahren zur Weiterverarbeitung von Gärrest aus Biogasanlagen zu Düngemitteln und eine Einrichtung zur Realisierung dieses Verfahrens bekannt gemacht. Obwohl der Ausgangspunkt dieses Verfahrens in Form der Fermentationsrückstände bestehender Biogasanlagen mit der beanspruchten technischen Lösung identisch ist, geht es tatsächlich nicht um das Gewinnen von Düngemitteln, sondern um die Qualitätsverbesserung der Fermentationsrückstände, die überwiegend bereits den Status von Wirtschaftsdüngern besitzen. Die Gewinnung von qualitätsverbesserten Düngemitteln gelingt vorschlagsgemäß möglicherweise energetisch günstiger als mit bekannten Verdampferlösungen. Keinesfalls ist jedoch mit einem Energiegewinn aus der Umsetzung von Teilen des in den Fermentationsrückständen enthaltenen Kohlenstoffinventars zu rechnen.

Die DE 10 2010 005 253 A1 beschreibt ein Verfahren und eine Vorrichtung zum Behandeln und/oder Aufbereiten von flüssigem Gärrest aus einem Nachgärer und/oder Fermenter einer Biogasanlage. Der Vorschlag enthält Maßnahmen zur Erhöhung des Energiegewinns aus dem mit den Einsatzstoffen einer bestehenden Methanfermentationsanlage zugeführten Kohlenstoff, um energetisch vorteilhafter die anfallenden Fermentationsrückstände trocknen und qualifiziert aufbereiten zu können. Als eine geeignete Maßnahme zur stofflichen und energetischen Verwertung der Fermentationsrückstände lassen sich diese Vorschläge dagegen nicht bewerten.

Die DE 10 2012 106 610 A1 offenbart ein Verfahren zur Herstellung von Langzeitdünger aus Gärresten mittels Verkapselung. Die vorgeschlagene technische Lösung beschränkt sich dabei auf die Gewinnung eines wirksamen organischen Düngemittels aus den Fermentationsrückständen einer Methanfermentation durch ergänzende Aufschluss-, Vermischungs- und stoffliche Zusatzmaßnahmen, nicht jedoch auf einen zusätzlichen Energiegewinn aus dem reichlich in den Fermentationsrückständen enthaltenen Kohlenstoffinventar.

Mit der DE 10 2012 112 989 A1 wird ein Verfahren und eine Anlage zur Herstellung von Biogas aus lignocellulosehaltiger Biomasse bekannt gemacht, mit dessen Hilfe rohfaserreiche und überwiegend lignifizierte Pflanzenreste, insbesondere Getreidestroh, der Methanfermentation zugänglich gemacht werden sollen. Dazu soll die verfügbare Biomasse mit einer wässrigen Flüssigkeit angemaischt und anschließend einem kurzzeitigen Kochprozess bei Temperaturen von wenigstens 130 °C unterworfen werden. Möglicherweise kommt es dabei zu ähnlichen Trennungseffekten von Cellulosefasern und der Ligninmatrix, wie sie aus dem in der Holzchemie für die Holzverzuckerung bekannten Dampfexplosionsverfahren bekannt sind. Da für die Methanfermentation Prozesstemperaturen von <70 °C benötigt werden, lässt sich diese technische Lösung nicht ohne zusätzliche energieaufwändige Heiz- und Kühlprozesse realisieren.

Die DE 10 2013 217 080 B4 beschreibt ein Düngepellet und ein Verfahren zu seiner Herstellung. Der Schwerpunkt dieser technischen Lösung bezieht sich auf das Bereitstellen einer phosphorreichen Biokohle, um schnelle Auswaschungen der enthaltenen Pflanzennährstoffe in den Wasserkreislauf zu verhindernd und die Transportwürdigkeit der pelletierten Düngemittel zu verbessern. Im Falle des Zusatzes von phophorhaltigen Schlämmen sollen dabei trockensubstanzreiche Fermentationsrückstände als Trocknungsmittel genutzt werden können.

Die EP 2 985 339 B1 macht einen Gärrest-Konditionierer und ein Verfahren zur Konditionierung von Gärresten bekannt. Obwohl sich die vorgeschlagene technische Lösung nicht auf die Verwertung von Rückständen der Methanfermentation, sondern auf die Behandlung von kommunalen Bioabfällen bezieht, sind bereits Hinweise auf den Einsatz von speziellen Mikrokulturen, auf die Nutzung von enzymatischen Hydrolyseschritten wenigstens für Teile des zu behandelnden Stoffstroms und die Behandlung der verflüssigten Biomassen im mesophilen und im thermophilen Milieu beschrieben.

Die DE 10 2015 210 871 A1 benennt ein Verfahren zur stofflichen Nutzung organischen Substrates. Die Autoren dieses Vorschlages kommen zu einem bemerkenswerten Ergebnis, indem die bekannte Unmöglichkeit der simultanen Realisierung der für einen effizienten Methanfermentationsprozess erforderlichen Prozessschritte in einem einheitlichen Prozessbehälter zum erklärten Verfahrensziel stilisiert wird. Wesentliches Verfahrensziel der vorgeschlagenen technischen Lösung ist danach neben der Methanisierung ohne eine räumliche Trennung unverzichtbarer Verfahrensschritte das Gewinnen eines unvollständig zu Biogas oder Methan abgebautes organisches Substrat. Ein solches Substrat soll danach ausdrücklich einem weiteren Fermentationsprozess zugeführt werden.

Den bekannten technischen Lösungen haftet der gemeinsame Mangel an, dass offenbar eine stoffliche und energetische Verwertung der verfügbaren Biomassen in Form von Fermentationsrückständen bisher nicht für möglich gehalten wird. Eine technische Lösung, mit der überwiegend auf an sich bekannte sanfte biotechnologische Prozeduren zurückgegriffen wird, ist bisher noch nicht offenbart worden.

### AUFGABE DER ERFINDUNG

Die Aufgabe der Erfindung besteht deshalb in der Überwindung der Mängel des bekannten Standes der Technik. Insbesondere soll eine technische Lösung bereitgestellt werden, mit deren Hilfe das in den Fermentationsrückständen aus konventionell betriebenen Biogasanlagen zur Verarbeitung von nachwachsenden Rohstoffen enthaltene energetische Potential angemessen genutzt und das in den Fermentationsrückständen enthaltene Potential an Pflanzennährstoffen nicht nur vollständig erhalten, sondern noch hinsichtlich der Transport- und Anwendungseigenschaften aufgewertet werden kann.

### LÖSUNG

Die Aufgabe der Erfindung wird gelöst durch ein Verfahren zur stofflichen und energetischen Verwertung von insbesondere festen Fermentationsrückständen aus der Methanfermentation von Energiepflanzen.

In einem ersten Schritt wird zumindest aus den Fermentationsrückständen in einem Suspensionsprozess unter Zugabe von Prozessflüssigkeit eine für die Nassfermentation geeignete Biosuspension, die demnach einen Trockensubtanzgehalt von nicht mehr als 15 % aufweist, hergestellt. In einem zweiten Schritt erfolgt eine hydrolytische Vorbehandlung, während der langkettige Verbindungen in der Biosuspension aufgebrochen werden. Und in einem dritten Schritt erfolgt eine vorzugsweise mehrstufige anaerobe Methanbildung, während der das Biogas mit seinem Hauptbestandteil Methan gebildet wird. Dies erfolgt in von der hydrolytischen Vorbehandlung räumlich getrennten Prozessbehältern.

Die Prozessbehälter sind kulturerhaltend ausgestattet oder werden kulturerhaltend betrieben, sodass die Mikrokultur, die Bakteriengruppen, die an den verschiedenen Prozessen teilnehmen, in Qualität und Quantität stabil bleibt. Im Ergebnis werden zumindest ein energetisch verwertbares methanhaltiges Biogas und ein stofflich verwertbares Düngemittel gebildet.

Nach der Erfindung weisen die Fermentationsrückstände einen Trockenmassegehalt von wenigstens 25 % auf und gelten damit als feste Fermentationsrückstände. Die dem Gemisch zugeführten festen Fermentationsrückstände tragen einen Anteil an Trockenmasse zwischen 40 % und 100 % der Trockenmasse des Gemischs bei. Die hydrolytische Vorbehandlung unter Einsatz von Sauerstoff wird entweder mittels Intensivrotte des feuchten und schüttgutförmigen Gemischs vor dem Suspensionsprozess und/oder durch Eintrag von Sauerstoff in die Biosuspension vorgenommen. Bei der Herstellung der Biosuspension werden Filtrate und/oder Kondensate als Suspendierungsmittel eingesetzt, die zumindest aus der Aufbereitung der Rückstände der nachfolgenden Methanbildung stammen und von dort zugeführt werden. Damit werden diese genutzt und eine gesonderte Entsorgung ist nicht erforderlich.

Beim Einsatz der Intensivrotte für die aerobe hydrolytische Vorbehandlung des feuchten und schüttgutförmigen Gemischs unter unlimitiertem Sauerstoffzutritt beträgt die Behandlungszeit zwischen 0,5 und 48 Stunden, vorzugsweise zwischen 1,0 und 3,0 Stunden. Die aerobe hydrolytische Vorbehandlung der Biosuspension wird räumlich von der Methanbildung getrennt und unter Zusatz von 0,6 bis 1,4 m³ Sauerstoff je kg zu hydrolysierender organischer Trockensubstanz, vorzugsweise im Verhältnis zwischen 0,8 bis 1,2 m³ Sauerstoff je kg hydrolysierender organischer Trockensubstanz, vorgenommen.

Nach einer alternativen Ausgestaltung des Verfahrens in einem der Herstellung der Biosuspension vorgelagerten Schritt wird ein Gemisch der festen Fermentationsrückstände mit tierischen Exkrementen und/oder organischen Reststoffen hergestellt und das Gemisch der Intensivrotte und/oder dem Suspensionsprozess zugeführt.

Es hat sich als vorteilhaft erwiesen, wenn sowohl die zur Herstellung des Gemischs eingesetzten tierischen Exkremente als auch die organischen Reststoffe wenigstens 28 % Trockensubstanzgehalt aufweisen. Besonders vorteilhaft ist es, wenn die Pflanzenreste rohfaserreich, da die Fasern u. a. als Aufwuchsfläche der Mikrokultur dienen können, und/oder harnsäurereich sind.

Insbesondere sind die organischen Reststoffe zumindest ausgewählt aus Pflanzenresten, Trester aus der Früchteverarbeitung und/oder Braureststoffen aus der Bierherstellung. Diese Stoffe können einzeln oder gemeinsam in unterschiedlicher Zusammensetzung als Bestandteile der Einsatzstoffe Anwendung finden.

Bei der hydrolytischen Behandlung der Biosuspension stammt der erforderliche Sauerstoff aus der Umgebungsluft oder aus Abluft oder es wird alternativ technischer Sauerstoff eingesetzt, insbesondere aus einer Gasflasche.

Es hat sich als vorteilhaft erwiesen, wenn sauerstoffhaltige Hydrolysegase aus der hydrolytischen Vorbehandlung direkt einer biologischen Gasentschwefelung zugeführt werden. Da die biologische Gasentschwefelung stets Sauerstoff für den Betrieb benötigt, ergibt sich aus der Zuführung der sauerstoffhaltigen Hydrolysegase ein Synergieeffekt, indem einerseits Sauerstoff zugeführt wird und andererseits eine Neutralisierung, insbesondere eine Geruchsminderung, der Hydrolysegase erfolgt.

Nach einer vorteilhaften Ausgestaltung des Verfahrens erfolgt die biologische Gasentschwefelung räumlich von der anaeroben Methanbildung getrennt. Dadurch kann der zur biologischen Gasentschwefelung erforderliche Sauerstoffeinsatz optimiert werden und beeinträchtigt nicht den anaeroben Prozess der Methanbildung.

Vorzugsweise erfolgt die aerobe hydrolytische Vorbehandlung der Biosuspension in einem begasbaren Behälter und/oder in einem begasbaren Förderaggregat und wird wenigstens für die erforderliche Dauer der Sauerstoffzufuhr aufrechterhalten. Die in der aeroben hydrolytischen Vorbehandlung erzeugte Hydrolysate werden einer ersten Stufe der anaeroben Methanbildung zugeführt und dort mit einer an mesophiles Milieu adaptierten Mikrokultur unter mesophilem Milieu behandelt. Der Prozess der Methanisierung läuft demnach unter mesophilen Bedingungen bei Temperaturen über 25 °C, jedoch unter 40 °C ab.

Es hat sich als vorteilhaft erwiesen, wenn zum Start der anaeroben Methanbildung einer Methanfermenteranordnung als eine biologische Erstausstattung Fermentationsrückstände einer Biogasanlage mit überwiegend rohfaserreichen Einsatzstoffen, beispielsweise Getreidestroh, als bereits adaptierte Mikrokultur eingesetzt werden. Eine solche Mikrokultur kann sich unter vergleichbaren Bedingungen schnell vermehren, ohne dass langwierige Adaptionsprozesse abgewartet und unterstützt werden müssten. Die Biogasproduktion setzt dadurch stabil und zügig ein.

Bevorzugt weist die zweite Stufe der anaeroben Methanbildung in einer Nachfermenteranordnung ein thermophiles Milieu auf. In der zweiten Stufe wird das die erste Stufe der anaeroben Methanbildung verlassende Fermentationssubstrat behandelt. Die dort aktive Mikrokultur ist an ein thermophiles Milieu adaptierten, mit dieser wird das Fermentationssubstrat behandelt.

Nach einer vorteilhaften Ausgestaltung werden die Nachfermenteranordnung der zweiten Stufe der anaeroben Methanbildung batchweise und bei mehr als einem Methanfermenter diese zusätzlich in wechselsweisem Prozessablauf betrieben. Der batchweise Betrieb benötigt nur ein Mindestmaß an Anlagentechnik, sodass die Nachfermentierung in einer technisch einfachen Anordnung erfolgen kann. Eine bevorzugte Mindestaufenthaltszeit der Fermentationssubstrate, die bei der batchweisen anaeroben Methanbildung in jedem Methanfermenter vor der teilweisen Entleerung aufrechterhalten wird, beträgt wenigstens 10 Tage.

Es hat sich als vorteilhaft erwiesen, wenn die anaerobe Methanbildung der zweiten Stufe in wenigstens einem Prozessbehälter mit kreisringförmiger Grundfläche als Nachfermenteranordnung erfolgt. Die in der Nachfermenteranordnung der zweiten Stufe der anaeroben Methanbildung vorgesehenen Umwälzeinrichtungen werden ausschließlich zur intermittierenden Fluidisierung von entstehenden Sedimenten genutzt, damit sich eine festen, schwer zu entfernenden Schichten am Boden ausbilden.

Die mit der vorliegenden Erfindung entwickelte technische Lösung nutzt den aufgefundenen Zusammenhang, dass die mit bisher bekannten technischen Mitteln kaum für die Methanfermentation erschließbaren Rohfasergehalte für die weitere energetische Verwertung dennoch erschlossen werden können. Diese Rohfasergehalte definieren sich als eine unterschiedlich stark lignifizierte Biomasse in den verfügbaren Fermentationsrückständen aus vorheriger Behandlung in einer Biogasanlage oder weiteren Rückständen aus der Pflanzen- und Lebensmittelproduktion. Die erfindungsgemäße energetische Verwertung erfolgt unter einem vergleichsweise geringen Energieeinsatz. In der Folge lässt sich diese vorbehandelte Biomasse dann mit einem insgesamt deutlich höheren Energiegewinn mit an sich bekannten Mitteln der Methanfermentation unterziehen.

Auf diese Weise stehen der Methanfermentation als zusätzliche Einsatzstoffe nun neben den Fermentationsrückständen aus den konventionell betriebenen Biogasanlagen, vorzugsweise aus den Biogasanlagen zur Verarbeitung von nachwachsenden Rohstoffen, auch
- das bisher weitgehend für die Methanfermentation nicht genutzten Stroh aus der Baumwoll-, Getreide- und Ölsaatenproduktion mit überwiegend lignifizierten Rohfasergehalten in silierter Form,
- die Brau- und Brennereirückstände der Bier- und Ethanolherstellung mit überwiegenden Rohfaser- und Proteingehalten und auch
- die Pülpen aus der Stärkeherstellung mit ebenfalls überwiegenden Rohfaser- und Proteingehalten
zur Verfügung.

Überraschenderweise zeigte sich, dass bereits eine intensive und kurzzeitige aerobe Behandlung der Einsatzstoffe in Form eines ausreichend feuchten Schüttgutes oder in Form einer wässrigen Suspension ausreicht, um den beobachteten beginnenden Verrottungsprozess für die Methanfermentation nutzbar zu machen. Die Modifikationen dieser Vorbehandlung sind dabei abhängig sowohl von den betroffenen Einsatzstoffen als auch von den zu erreichenden Zielen der Methanfermentation.

In Abhängigkeit von den verfügbaren Einsatzstoffen und den Zielen der Bioenergiegewinnung mittels Methanfermentation können für die aerobe Vorbehandlung alternativ, parallel oder aufeinanderfolgend mehrere technische Lösungen genutzt werden.

Allgemein bekannt ist dabei die aerobe oder semi-aerobe Hydrolyse der aus den Einsatzstoffen und den recycelten Biofiltraten erzeugten Biosuspension vor dem Einsatz der dabei gewonnenen Hydrolysate in den mehrstufigen Methanisierungsprozess. Zur Vermeidung von Umweltbelastungen einerseits und von verfahrenstechnischen Schwierigkeiten andererseits erfordern die dabei anfallenden Hydrolysegase zusätzliche verfahrenstechnische Maßnahmen. Infolge des Einsatzes von ammoniumhaltigen Biofiltraten als Suspendierungsmittel kommt es bei der hydrolytischen Behandlung der Biosupensionen bei Temperaturen von bis zu 70 °C zur beachtlichen Ammoniakentbindung, wodurch die energetische Direktnutzung bereits von Anteilen der anfallenden unbehandelten Hydrolysegase im Kessel- oder Motorenbetrieb zu erhöhten NOₓ-Emissionen und zugleich zu Stickstoffverlusten für die Verwertung der Pflanzennährstoffe führt. Die für die hydrolytische Vorbehandlung der suspendierten rohfaserreichen Einsatzstoffe benötigten Sauerstoffmengen übersteigen regelmäßig bei der bevorzugte biologischen Gasentschwefelung aus Gründen des Explosionsschutzes das zulässige Maß, so dass nur Anteile des Hydrolysegases den Rohgasen aus dem mehrstufigen Fermentationsprozess vor der Gasentschwefelung zugeführt werden können. Dagegen kann ein Anteil von molekularem Stickstoff bei Einsatz von Luft als Sauerstoffquelle für den aeroben Hydrolyseprozess durch die Verwendung von technischem Sauerstoff vermieden werden, wenn dies die anschließende Aufbereitung des entschwefelten Biogases zu Bioerdgas (auch als Biomethan bezeichnet) erschwert.

Grundsätzlich vorteilhaft ist dagegen der Einsatz der rohfaserreichen biogenen Einsatzstoffe in Form ausreichend feuchten Schüttgutes, weil für deren aerobe Vorbehandlung die an sich bekannten Techniken der Intensivrotte nutzbar sind. Rohfaserreiche und zugleich proteinhaltige biogene Einsatzstoffe, wie Strohsilagen und Presskuchen aus Biertreber, Ethanolschlempen und Stärkepülpen lassen sich dabei aerob behandeln, ohne die bekannten Rottegase aus lediglich periodisch umgewälzten Rottemieten, wie Methan, Lachgas und dergleichen zu emittieren. Bevorzugt wird die Intensivrotte in beheizbaren und belüfteten Rottetrommeln oder in großvolumigen und für die Kontakttrocknung gebräuchlichen Förderschnecken realisiert.

Die Rottegase aus den geschlossenen und beheizbaren Apparaten für die Intensivrotte werden umweltgerecht über übliche und bekannte Biofilter in die Atmosphäre geleitet, wobei im Falle der eingesetzten Fermentationsreste wegen der darin immer enthaltenen Ammoniumgehalte die entbundenen Ammoniakmengen den Biofiltern zur Vermeidung von Umweltschäden und Nährstoffverlusten eine schwefelsaure Wäsche mit der Gewinnung von wässriger Ammoniumsulfatlösung vorgeschaltet werden soll.

Erfindungsgemäß werden die im Durchlaufverfahren oder batchweise betriebenen Rotteapparate immer in die Suspensionsanordnung entleert, um Biosuspensionen herzustellen und dem Fermentationsprozess zuzuführen, die ausnahmslos frische Einsatzstoffe enthalten, die einer wirksamen aeroben Vorbehandlung unterzogen wurden. Zugleich werden auf diese Weise dem Fermentationsprozess weder Stickstoff noch Sauerstoff zugeführt.

Die Vorteile der erfindungsgemäßen technischen Lösung gegenüber dem Stand der Technik lassen sich wie folgt zusammenfassen:
- Mit der entwickelten verfahrenstechnischen Lösung ist es möglich, flächendeckend in den Standortregionen mit bestehenden Methanfermentationsanlagen (Biogasanlagen) die festen Anteile der dort anfallenden Fermentationsrückstände unmittelbar zu übernehmen und weiter zu verarbeiten.
- Damit wird eine bereits erschlossene regenerative Energiequelle genutzt, ohne dafür nochmals landwirtschaftliche Nutzflächen oder Futter- und/oder Lebensmittel als Biomassequelle in Anspruch nehmen zu müssen.
- Die in den eingesetzten Fermentationsrückständen enthaltenen Pflanzennährstoffe bleiben für den Anlieferer oder für den Düngemittelhandel vollständig erhalten und werden hinsichtlich ihrer Transportwürdigkeit und ihrer Einsatzanforderungen spürbar aufgewertet in den Wirtschaftskreislauf zurückgeführt.
- Die biotechnologische Folgenutzung der eingesetzten Fermentationsrückstände wird auch dadurch ermöglicht, dass die Einsatzstoffe selbst von verschiedenen Lieferanten kaum wesentliche Qualitätsunterschiede aufweisen. Insbesondere betrifft dies die biotechnologische Vorbehandlung und die weitgehende Freiheit von leicht durch die verfügbaren Mikrokulturen angreifbaren Bestandteile in Form von Rohfetten, Rohproteinen und einfachen Kohlenwasserstoffen.
- Durch die nahezu vollständige Unabhängigkeit der vorgeschlagenen technischen Lösung von Roh- und Hilfsstoffimporten und von fossilen Prozessenergien in jeglicher Form ist die Bioenergiebereitstellung und die Bereitstellung qualitätsverbesserter organischer Düngemittel im besten Sinne nachhaltig.
- Da über die in geschlossenen Hallen zur Überbrückung von Störungen im Logistikbereich bevorrateten Einsatzstoffe und organische Düngemittel hinaus keine Lager betrieben werden müssen, wird eine beispielhafte Emissionsfreiheit von Schadstoffen für die Schutzgüter erreicht.
- Die erfindungsgemäße technische Lösung besitzt wegen des weitgehenden Entfalls mechanischer Medienumwälzungen in den anaerob betriebenen Prozessbehältern, wegen des teilweise selbsttätigen Transports der Fermentationssubstrate zwischen den anaerob betriebenen Prozessbehältern und wegen des ermöglichten Verzichtes auf den Einsatz von mechanischen Mitteln für den Gastransport aus den nicht flexibel abgedeckten Prozessbehältern einen minimierten elektrischen Prozessenergiebedarf.
   Wegen
- des Verzichtes auf den Eintrag der Fermentationsgifte Sauerstoff und Ammoniak in die Fermentationsbehälter,
- wegen der sicheren Gewährleistung der erforderlichen Mindestbehandlungszeiten im anaeroben Milieu,
- wegen des Erhalts der an die mittlere Qualität der zu verwertenden biogenen Einsatzstoffe zunehmend besser adaptierten Mikrokulturen durch die Nutzung von kulturerhaltender Fermentertechnik in der Hauptfermentationsstufe und der kulturerhaltenden Betriebsweise der im Batchbetrieb genutzten Fermentationsbehälter in der Nachgärstufe und
- wegen des weitgehenden Ausschlusses von mechanischen Scherkräften beim Umwälzen der Gärsubstrate in den Fermentationsbehältern durch die Werkzeuge von Rührmechanismen
   werden maximale spezifische Biogaserträge erzielt.
- Der bewirkte zusätzliche Abbau des mit den Fermentationsrückständen in den neuerlichen Fermentationsprozess eingetragenen Kohlenstoffs führt zur weiteren Absenkung des Anteils der organischen Substanz an der Trockensubstanz der Rückstände der verfahrensgemäßen Methanfermentation, womit der spezifische Gehalt der mineralischen Pflanzennährstoffe in den Fermentationsrückständen spürbar erhöht wird.
- Die Emission von Wasserschadstoffen, als die die beachtlichen Ammoniumgehalte in den Fermentationssubstraten gelten, ist durch die 100 %-ige Doppelwandigkeit der Prozessbehälter und Rohrleitungen mit der Aufnahmefähigkeit der abflusslosen und mit einer umlaufenden Aufkantung ausgestatteten betonierten Standortfläche ausgeschlossen.
- Die Emission von gasförmigen Klimaschadstoffen ist auf die Abgasemission der zur Gewährleistung der energieautarken Prozessführung genutzten Gasmotoren- und/oder Gaskesseltechnik begrenzt.
- Die vollständig geschlossene Bauweise der zusammengeschalteten Prozessbehälter verhindert sicher Geruchsemissionen als Voraussetzung für die Anlagenpositionierung in der Nähe von Wohngebäuden.

### AUSFÜHRUNGSBEISPIELE

Die Erfindung soll nachstehend mit Ausführungsbeispielen näher erläutert werden. In der beigefügten Zeichnung zeigen
Fig. 1 die schematische Darstellung der Verknüpfung der erforderlichen Verfahrensschritte für eine Bioenergieanlage, die ausschließlich fermentierten und andere Wirtschaftsdünger verwertet;
Fig. 2 die schematische Darstellung der Verknüpfung der erforderlichen Verfahrensschritte für eine Bioenergieanlage zur Verwertung von Fermentationsrückständen und rohfaserreichen Pflanzenresten; und
Fig. 3 die schematische Darstellung der Verknüpfung der erforderlichen Verfahrensschritte für eine Bioenergieanlage zur Verwertung von Fermentationsrückständen und rohfaserreichen Rückständen der Lebensmittel- und Getränkeindustrie.

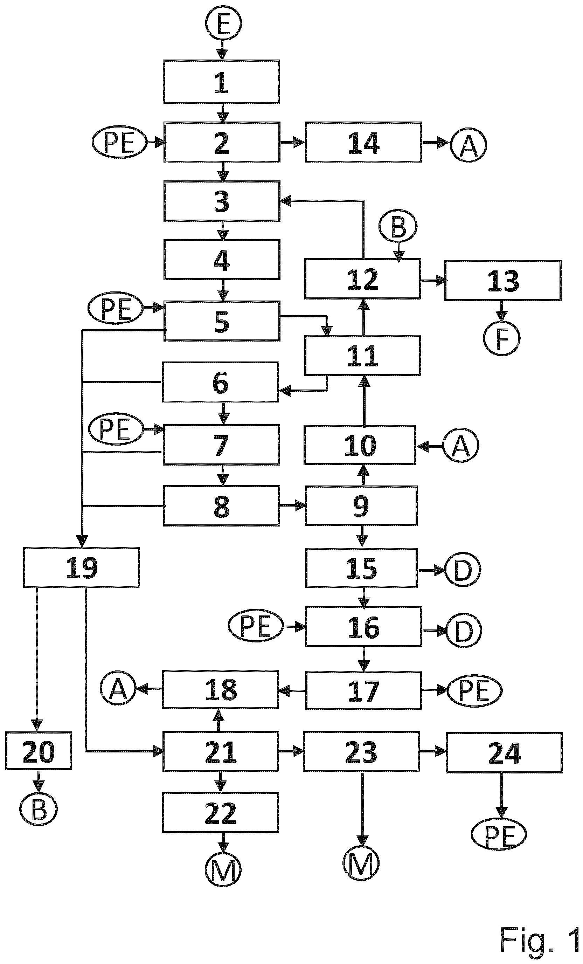

**Beispiel 1:** Gemäß der Fig. 1 stehen in der Standortregion zum Betrieb einer Bioenergieanlage Fermentationsrückstände von verschiedenen konventionellen Anlagen zur Gewinnung von Biogas aus der so genannten Anbaubiomasse, wie Ganzpflanzensilage und Feldfrüchten, zur Verfügung. Zusätzlich sollen rohfaserreiche Miste aus der Tierproduktion stofflich und energetisch verwertet werden.

Die Anlage, die das erfindungsgemäße Verfahren nutzt, besteht dabei aus einer ersten Mühlenanordnung 1, vorzugsweise einem Querstromzerspaner, für die rohfaserreichen Festmiste, tierische Exkremente mit hohem Trockensubstanzgehalt. Sowohl der Austrag der ersten Mühlenanordnung 1 als auch die einzusetzenden feinteiligen Fermentationsrückstände gelangen direkt in die Intensivrotteanordnung 2, die in Form einer großvolumigen belüfteten und beheizbaren Rottetrommel ausgebildet ist. Die Rottetrommel ist auf der Decke der Suspensionsanordnung 3 positioniert und kann stetig oder periodisch in die Suspensionsanordnung 3 entleert werden. Das von der Intensivrotteanordnung 2 abgesaugte Rottegas gelangt zur Nachbehandlung in die Biofilteranordnung 14, die wegen des Einsatzes der ammoniumhaltigen Fermentationsreste mit einem vorgeschalteten schwefelsauer betriebenen Gaswäscher zur Ammoniakbindung ausgestattet ist. Die in der Suspensionsanordnung 3 unter Einsatz des in der Hemmstoffentfrachtungsanordnung 12 teilweise von Ammonium entfrachteten keimhaltigen Biofiltrats erzeugte Biosuspension gelangt über die zweite Mühlenanordnung 4 für die Feinstzerkleinerung der festen Inhaltsstoffe der Suspension in die batchweise betriebenen Tanks der Hydrolyseanordnung 5. Dort wird unter Einsatz von Umgebungsluft der aerobe Vorbehandlungsprozess fortgesetzt, nun jedoch limitiert durch die maximal zulässige Sauerstoffbelastung der biologischen Gasentschwefelungsanordnung 19. Die Hydrolyseanordnung 5 dient zugleich als Entkopplungselement von der Suspensionsanordnung 3, wo die Herstellung der Suspension erfolgt, und der Methanfermenteranordnung 6. Die in der Hydrolyseanordnung 5 entstehenden Hydrolysegase werden unabhängig von den Biogasen aus den Fermentationsstufen 6, 7 und dem Gärrestdosiertank 8 direkt zur Gasentschwefelungsanordnung 19 geleitet. Die in den batchweise betriebenen Tanks der Hydrolyseanordnung 5 anfallenden Hydrolysate werden über den Hydrolysatkühler 11 zur Methanfermenteranordnung 6 geleitet, in der die eingesetzten Fermentationsapparate bevorzugt im mesophilen Milieu betrieben werden.

In der Methanfermenteranordnung 6 kommen kulturerhaltende und hydraulisch umwälzbare Großfermenter zum Einsatz. Durch den kulturerhaltenden Effekt wird eine Schädigung der Mikrokultur, die an der Methanbildung beteiligten Bakterien, vermieden. Dies steht nicht zuletzt in Zusammenhang mit dem hydraulischen Umwälzen. Dieses wird erreicht, indem infolge des Gasdrucks durch das gebildete methanhaltige Biogas in einem Behälter das Gärsubstrat in einen zweiten Behälter gedrückt wird und nachfolgend wieder zurückströmt. Hierdurch kommt es zum Umwälzen des Gärsubstrats. Das Umwälzen geschieht durch Verzicht auf gesonderte Rührwerke (z. B. Propellerrührwerke) mechanisch schonend und insbesondere ohne Scherkräfte in das Substrat einzuleiten. Dies trägt, neben konstanten Bedingungen, zur Kulturerhaltung bei.

Jeweils bei der regelmäßigen Beschickung der Methanfermenteranordnung 6 mit gekühltem Hydrolysat werden die aus den Apparaten der Methanfermenteranordnung 6 verdrängten Gärsubstrate in den jeweils aufnahmebereiten Fermentationsapparat der Nachfermenteranordnung 7 gefördert.

Die Fermentationsapparate der Nachfermenteranordnung 7 werden ebenfalls kulturerhaltend, jedoch batchweise bzw. diskontinuierlich, und im thermophilen Milieu betrieben. Zugleich wird die jeweilige Mindestbehandlungszeit des Gärsubstrates im thermophilen Milieu von wenigstens 10 Tagen im Interesse der Gewährleistung der Vollstrom-Hygienisierung der daraus gewinnbaren organischen Stickstoff-Phosphor-Kali-Schwefel-Dünger (NPKS-Dünger) für den Düngemittelhandel eingehalten. Bei der Hygienisierung werden pathogene Keime abgetötet, sodass ein hygienisch einwandfreier Dünger gewonnen werden kann, ohne dass weitere Maßnahmen erforderlich würden, um die Hygieneanforderungen zu erfüllen.

Der Inhalt des jeweils für die Hygienisierung ausreichend lange betriebenen Apparates der Nachfermenteranordnung 7 wird zur Erhaltung der genutzten Mikrokultur nur teilweise in den Gärrestdosiertank 8 umgepumpt, um immer einen aufnahmebereiten Methanfermenter mit einem ausreichend groß bemessenen Rest der vermehrungsfähigen adaptierten thermophilen Mikrokultur für die Aufnahme des Gärsubstrates aus der Methanfermenteranordnung 6 verfügbar zu haben. Dies trägt beim Batchverfahren zur Kulturerhaltung bei.

Die Gärreste werden aus dem Gärrestdosiertank 8 ununterbrochen der Phasentrennanordnung 9 zur physikalischen Fest-Flüssigtrennung der Gärreste zugeführt. Die nach Abscheiden der flüssigen Bestandteile, der Dünnphase, anfallenden Presskuchen gelangen unmittelbar als Schüttgut in das geschlossene Feuchtdüngerlager 15, während die Dünnphase als Biofiltrat im Biofiltratdosiertank 10 zwischengelagert wird.

Aus dem Biofiltrattank 10, in den auch die Einleitung von Abluft A aus der Biofilteranordnung 14 und dem Kondensattank 18 erfolgt, wird das ammoniumreiche Biofiltrat der Hemmstoffentfrachtungsanordnung 12 zugeführt. Zwischenzeitlich wird das ammoniumreiche Biofiltrat im Hydrolysatkühler 11 als Kühlmittel voraufgeheizt. In der Hemmstoffentfrachtungsanordnung 12 wird das Biofiltrat zur Vermeidung toxisch wirkender überhöhter Ammoniakgehalte im Fermentationssubstrat von Teilen des enthaltenen Ammoniums entfrachtet. Dabei werden unter Einsatz der in der Gasentschwefelungsanordnung 19 gewonnenen schwefligen Biosäure B Ammoniumsulfatlösungen gebildet. Diese werden im Ammoniumsulfattank 13 für die Nutzung als Flüssigdünger F zwischengelagert. Anteile der im Feuchtdüngerlager 15 anfallenden organischen NPKS-Düngemittel D (Düngemittel, das Stickstoff N, Phosphor P, Kalium K und Schwefel S - NPKS - enthält) werden zur Verbesserung der Transportwürdigkeit, ohne unnötige zusätzliche Last durch enthaltenes Wasser, der Düngertrocknungsanordnung 16 zugeführt.

Die Düngertrocknungsanordnung 16 wird bevorzugt mit Kontakttrocknungstechnik betrieben, um u. a. die anfallenden Trocknungswrasen in einem Wrasenkondensator 17 als Prozessflüssigkeit verflüssigen zu können. Die Prozessflüssigkeit wird ebenfalls zur Herstellung der Biosuspension verwendet. Bei diesem Prozess wird die Kondensationswärme für einen Teil der biotechnologischen Aufgaben als Prozessenergie PE zurückgewonnen.

Das entschwefelte Rohgas als Gemisch der Biogase aus den Fermentationsanordnungen 6, 7 und dem Gärrestdosiertank 8 sowie den lufthaltigen Hydrolysegasen aus der Hydrolyseanordnung 5 verlässt die Gasentschwefelungsanordnung 19 und gelangt in den Reingasspeicher 21, der als Doppelmembrangasspeicher auf dem Kondensattank 18 positioniert ist. Die in der Gasentschwefelungsanordnung 19 gewonnene schwefelsaure Biosäure B wird im Biosäuretank 20 gespeichert, um als Waschmedium für die ammoniakhaltigen Wrasen der Hemmstoffentfrachtungsanordnung 12 und der Düngertrocknungsanordnung 16 verfügbar zu sein.

Der Reingasspeicher 21 ist ausreichend groß dimensioniert, um die vorgesehene Entkopplungsfunktion von Rohgasbereitstellung und den nachgeschalteten Reingasverwertungen erfüllen zu können. Zur Reingasverwertung erfolgt die Gasaufbereitung 22 zu Biomethan (Methan biogenen Ursprungs) oder zur Gastrocknungs- und -verdichtungsanordnung 23 mit dem nachgeordneten Blockheizkraftwerk 24, das Elektroenergie und Wärme abgibt.

Die entscheidende verfahrenstechnische Wirkung der erfindungsgemäßen technischen Lösung wird bereits erreicht, wenn insbesondere die aerobe Vorbehandlung der feuchten Einsatzstoffe in der als Intensivrotteanordnung 2 gewählten Rottetrommel bei einer maximalen Rottetemperatur von 70 °C über wenigstens 3 Stunden aufrechterhalten wird. Eine verlängerte Rottedauer führt nicht zwingend zu einer Umkehr des Verhältnisses von Kohlenstoffentzug im Rotteprozess zur vergrößerten Kohlenstoff-Verfügbarkeit für die Methanisierung, beeinträchtigt jedoch die überraschend erkannten Vorteile der verbesserten Zugänglichkeit der Rohfasern in den Einsatzstoffen unter Inkaufnahme der Energieverluste aus dem initiierten Rotteprozess.

Die erzielbaren Prozesskennziffern sind für dieses Beispiel in der Tabelle 1 zusammengestellt:

**Tabelle 1: Kennwerte zum Beispiel 1**

| **Pos.** | **Bezeichnung** | **Dimension** | **Betrag** |
|---|---|---|---|
| 1. | Einsatzstoffe | | |
| 1.1 | Fermentationsrückstände | t/a | 360.000 |
| 1.2 | Mais-/Rapsstrohsilagen | t/a | - |
| 1.3 | Geflügelexkremente | t/a | 80.000 |
| 1.4 | Braureststoffe | t/a | - |
| 1.5 | Summe | t/a | 440.000 |
| 2. | Trockensubstanzmenge der Einsatzstoffe (TS) | t/a | 144.800 |
| 3. | org. Trockensubstanzmenge der Einsatzstoffe (oTS) | t/a | 128.120 |
| 4. | Verhältnis oTS/TS in den Einsatzstoffen | % TS | 88,5 |
| 5. | TS-Menge der erzeugten organischen NPKS-Düngemittel | t/a | 106.766 |
| 6. | in den Düngemitteln enthaltene oTS-Menge | t/a | 91.392 |
| 7. | Verhältnis oTS/TS in den organischen Düngemitteln | % TS | 85,6 |
| 8. | Erzeugtes Methanvolumen | Mio. m³/a | 45,25 |
| 9. | gewonnene thermische Energie | GWh/a | 452,5 |
| 10. | spezifischer Methanertrag | m³/kg oTS | 0,35 |

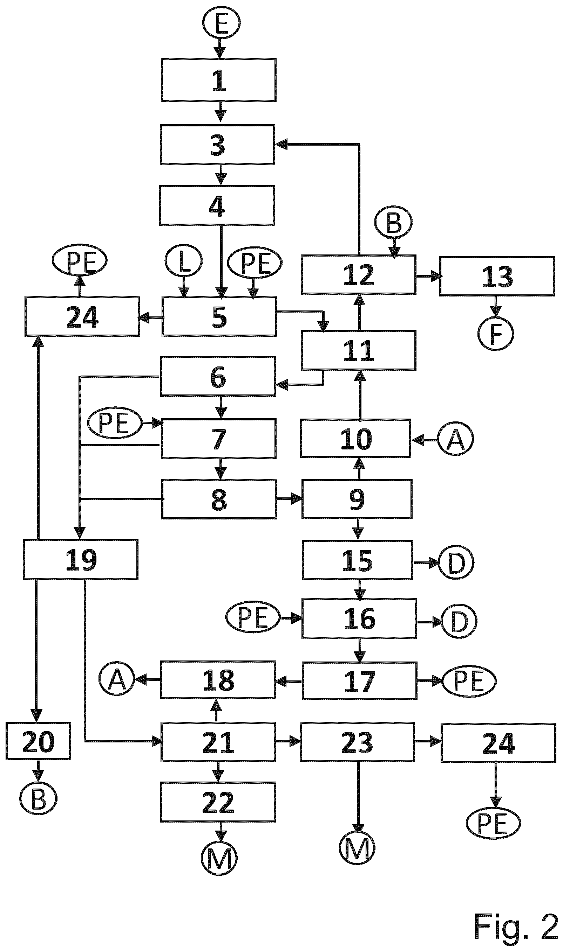

**Beispiel 2:** In diesem Beispiel unterscheidet sich gemäß Fig. 2 das Gemisch von Einsatzstoffen gegenüber dem Beispiel 1 dadurch, dass ausschließlich Fermentationsrückstände aus konventionell betriebenen Biogasanlagen, die der Verarbeitung von nachwachsenden Rohstoffen und Strohsilagen, vorzugsweise aus Maisstroh und/oder Rapsstroh, dienen, eingesetzt werden. Auf die Nutzung einer Intensivrotteanordnung 2 mit einer Nachbehandlung der Rottegase mittels Biofiltertechnik 14 wird verzichtet. Dafür wird im erforderlichen Maße die aerobe Behandlung der erzeugten Biosuspension in der Hydrolyseanordnung 5 verlängert. Die sauerstoffhaltigen Hydrolysegase werden dabei komplett unter Zusatz von entschwefeltem Biogas aus der Gasentschwefelungsanordnung 19 energetisch zur zusätzlichen Prozessenergiegewinnung, vorzugsweise in einem BHKW, eingesetzt.

**Tabelle 2: Kennwerte zum Beispiel 2**

| **Pos.** | **Bezeichnung** | **Dimension** | **Betrag** |
|---|---|---|---|
| 1. | Einsatzstoffe | | |
| 1.1 | Fermentationsrückstände | t/a | 190.000 |
| 1.2 | Mais-/Rapsstrohsilagen | t/a | 70.000 |
| 1.3 | Geflügelexkremente | t/a | - |
| 1.4 | Braureststoffe | t/a | - |
| 1.5 | Summe | t/a | 260.000 |
| 2. | Trockensubstanzmenge der Einsatzstoffe (TS) | t/a | 84.700 |
| 3. | org. Trockensubstanzmenge der Einsatzstoffe (oTS) | t/a | 76.230 |
| 4. | Verhältnis oTS/TS in den Einsatzstoffen | % TS | 90,0 |
| 5. | TS-Menge der erzeugten organischen NPKS-Düngemittel | t/a | 60.614 |
| 6. | in den Düngemitteln enthaltene oTS-Menge | t/a | 52.612 |
| 7. | Verhältnis oTS/TS in den organischen Düngemitteln | % TS | 86,8 |
| 8. | Erzeugtes Methanvolumen | Mio. m³/a | 28,16 |
| 9. | gewonnene thermische Energie | GWh/a | 281,6 |
| 10. | spezifischer Methanertrag | m³/kg oTS | 0,37 |

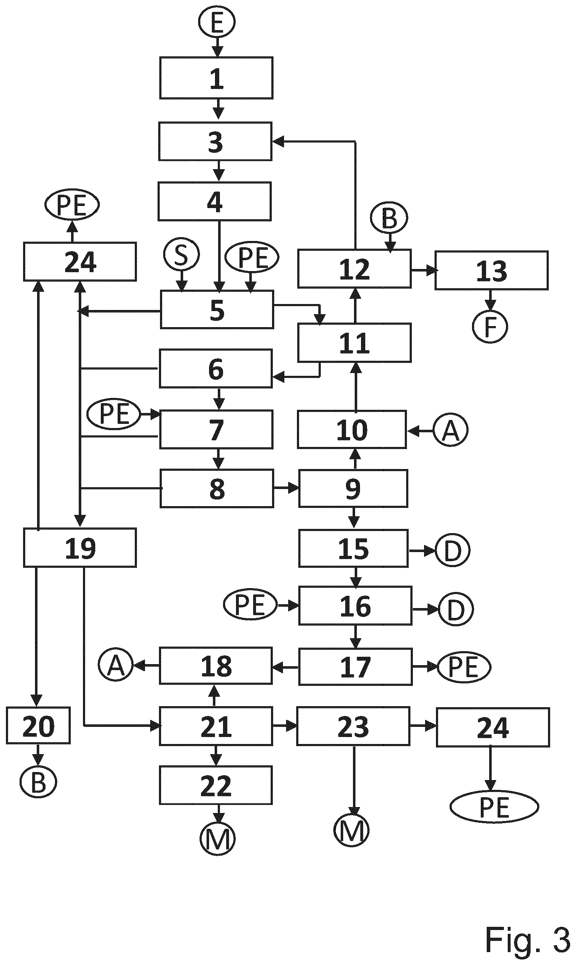

**Beispiel 3:** Im Beispiel 3 erfolgt die Behandlung eines Gemischs von Einsatzstoffen gemäß Fig. 3 dadurch, dass den Fermentationsresten aus den konventionell betriebenen Biogasanlagen, die der Verarbeitung von nachwachsenden Rohstoffen dienen, Trester aus der Früchteverarbeitung sowie Braureststoffe aus der Bierherstellung zugesetzt wurden. Anstelle von Umgebungsluft wird der hydrolytischen Vorbehandlung der erzeugten Biosuspension in der Hydrolyseanordnung 5 technischer Sauerstoff, der aus einer Druckgasflasche entnommen wird, zugeführt und durch einen begrenzten Anteil der Hydrolysegase in der Gasentschwefelungsanordnung 19 ein entschwefeltes Reingas bereitgestellt wird, das im Interesse der einfachen Gasaufbereitung 22 zu Bioerdgas bzw. Biomethan nicht durch molekularem Stickstoff belastet ist.

**Tabelle 3: Kennwerte zum Beispiel 3**

| **Pos.** | **Bezeichnung** | **Dimension** | **Betrag** |
|---|---|---|---|
| 1. | Einsatzstoffe | | |
| 1.1 | Fermentationsrückstände | t/a | 23.000 |
| 1.2 | Mais-/Rapsstrohsilagen | t/a | 77.000 |
| 1.3 | Geflügelexkremente | t/a | - |
| 1.4 | Braureststoffe | t/a | 62.000 |
| 1.5 | Summe | t/a | 162.000 |
| 2. | Trockensubstanzmenge der Einsatzstoffe (TS) | t/a | 54.723 |
| 3. | org. Trockensubstanzmenge der Einsatzstoffe (oTS) | t/a | 50.024 |
| 4. | Verhältnis oTS/TS in den Einsatzstoffen | % TS | 91,4 |
| 5. | TS-Menge der erzeugten organischen NPKS-Düngemittel | t/a | 38.340 |
| 6. | in den Düngemitteln enthaltene oTS-Menge | t/a | 33.624 |
| 7. | Verhältnis oTS/TS in den organischen Düngemitteln | % TS | 87,7 |
| 8. | Erzeugtes Methanvolumen | Mio. m³/a | 19,74 |
| 9. | gewonnene thermische Energie | GWh/a | 197,2 |
| 10. | spezifischer Methanertrag | m³/kg oTS | 0,395 |

### Bezugszeichenliste

- 1: Mühlenanordnung I
- 2: Intensivrotteanordnung
- 3: Suspensionsanordnung
- 4: Mühlenanordnung II
- 5: Hydrolyseanordnung
- 6: Methanfermenteranordnung
- 7: Nachfermenteranordnung (mit Rohgasspeicher)
- 8: Gärrestdosiertank
- 9: Phasentrennanordnung
- 10: Biofiltratdosiertank
- 11: Hydrolysatkühler
- 12: Hemmstoffentfrachtungsanordnung
- 13: Ammoniumsulfattank
- 14: Biofilteranordnung
- 15: Feuchtdüngerlager
- 16: Düngertrocknungsanordnung
- 17: Wrasenkondensator
- 18: Kondensattank
- 19: Gasentschwefelungsanordnung
- 20: Biosäuretank
- 21: Reingasspeicher
- 22: Gasaufbereitung
- 23: Gastrocknungs- und -verdichtungsanordnung
- 24: Blockheizkraftwerk
- A: Abluft
- B: Biosäure
- D: Düngemittel (feucht, NPKS)
- E: Einsatzstoffe
- F: Flüssigdünger
- L: Luft
- M: (Bio-)Methan
- PE: Prozessenergie
- S: Sauerstoff

## Patentansprüche

**1.** Verfahren zur stofflichen und energetischen Verwertung von Fermentationsrückständen aus der Methanfermentation von Energiepflanzen, wobei in einem ersten Schritt zumindest aus den Fermentationsrückständen in einem Suspensionsprozess unter Zugabe von Prozessflüssigkeit eine für die Nassfermentation geeignete Biosuspension hergestellt wird, in einem zweiten Schritt eine hydrolytische Vorbehandlung und in einem dritten Schritt eine anaerobe Methanbildung in von der hydrolytischen Vorbehandlung räumlich getrennten Prozessbehältern erfolgen, wobei die Prozessbehälter kulturerhaltend ausgestattet oder kulturerhaltend betrieben werden, sodass die Mikrokultur in Qualität und Quantität stabil bleibt, wobei zumindest ein energetisch verwertbares methanhaltiges Biogas und ein stofflich verwertbares Düngemittel gebildet werden, **dadurch gekennzeichnet,**
**dass** die Fermentationsrückstände einen Trockenmassegehalt von wenigstens 25 % aufweisen und damit als feste Fermentationsrückstände gelten,
**dass** die dem Gemisch zugeführten festen Fermentationsrückstände einen Anteil an Trockenmasse zwischen 40 % und 100 % der Trockenmasse des Gemischs beitragen,
**dass** die hydrolytische Vorbehandlung unter Einsatz von Sauerstoff entweder mittels Intensivrotte des feuchten und schüttgutförmigen Gemischs vor dem Suspensionsprozess und/oder durch Eintrag von Sauerstoff in die Biosuspension vorgenommen wird,
**dass** bei der Herstellung der Biosuspension Filtrate und/oder Kondensate als Suspendierungsmittel eingesetzt werden, die zumindest aus der Aufbereitung der Rückstände der nachfolgenden Methanbildung stammen und von dort zugeführt werden,
**dass** beim Einsatz der Intensivrotte für die aerobe hydrolytische Vorbehandlung des feuchten und schüttgutförmigen Gemischs unter unlimitiertem Sauerstoffzutritt die Behandlungszeit zwischen 0,5 und 48 Stunden, vorzugsweise zwischen 1,0 und 3,0 Stunden, beträgt, und
**dass** die aerobe hydrolytische Vorbehandlung der Biosuspension räumlich von der Methanbildung getrennt und unter Zusatz von 0,6 bis 1,4 m³ Sauerstoff je kg zu hydrolysierender organischer Trockensubstanz, vorzugsweise im Verhältnis zwischen 0,8 bis 1,2 m³ Sauerstoff je kg hydrolysierender organischer Trockensubstanz, vorgenommen wird.

**2.** Verfahren nach dem Anspruch 1, wobei in einem der Herstellung der Biosuspension vorgelagerten Schritt ein Gemisch der festen Fermentationsrückstände mit tierischen Exkrementen und/oder organischen Reststoffen hergestellt und das Gemisch der Intensivrotte und/oder dem Suspensionsprozess zugeführt wird.

**3.** Verfahren nach dem Anspruch 2, wobei die zur Herstellung des Gemischs eingesetzten tierischen Exkremente und organischen Reststoffe wenigstens 28 % Trockensubstanzgehalt aufweisen, und wobei die Pflanzenreste rohfaserreich und/oder harnsäurereich sind.

**3.** Verfahren nach Anspruch 2 oder 3, wobei die organischen Reststoffe zumindest ausgewählt sind aus Pflanzenresten, Trester aus der Früchteverarbeitung und/oder Braureststoffen aus der Bierherstellung.

**4.** Verfahren nach einem der vorgenannten Ansprüche, wobei der bei der hydrolytischen Behandlung der Biosuspension erforderliche Sauerstoff aus der Umgebungsluft oder aus Abluft stammt oder technischer Sauerstoff eingesetzt wird.

**5.** Verfahren nach einem der vorgenannten Ansprüche, wobei sauerstoffhaltige Hydrolysegase aus der hydrolytischen Vorbehandlung direkt einer biologischen Gasentschwefelung zugeführt werden.

**6.** Verfahren nach Anspruch 5, wobei die biologische Gasentschwefelung räumlich von der anaeroben Methanbildung getrennt erfolgt.

**7.** Verfahren nach einem der vorgenannten Ansprüche, wobei die aerobe hydrolytische Vorbehandlung der Biosuspension in einem begasbaren Behälter und/oder in einem begasbaren Förderaggregat erfolgt und wenigstens für die erforderliche Dauer der Sauerstoffzufuhr aufrechterhalten wird.

**8.** Verfahren nach einem der vorgenannten Ansprüche, wobei bei der aeroben hydrolytischen Vorbehandlung erzeugte Hydrolysate einer ersten Stufe der anaeroben Methanbildung zugeführt und dort mit einer an mesophiles Milieu adaptierten Mikrokultur unter mesophilem Milieu behandelt werden.

**9.** Verfahren nach einem der vorgenannten Ansprüche, wobei zum Start der anaeroben Methanbildung einer Methanfermenteranordnung (6) als eine biologische Erstausstattung Fermentationsrückstände einer Biogasanlage mit überwiegend rohfaserreichen Einsatzstoffen als bereits adaptierte Mikrokultur die eingesetzt werden.

**10.** Verfahren nach einem der vorgenannten Ansprüche, wobei die zweite Stufe der anaeroben Methanbildung in einer Nachfermenteranordnung (7), in der das die erste Stufe der anaeroben Methanbildung verlassende Fermentationssubstrat behandelt wird, ein thermophiles Milieu aufweist und mit einer an thermophiles Milieu adaptierten Mikrokultur behandelt werden.

**11.** Verfahren nach einem der vorgenannten Ansprüche, wobei die Nachfermenteranordnung (7) der zweiten Stufe der anaeroben Methanbildung batchweise und bei mehr als einem Methanfermenter diese zusätzlich in einem wechselsweisen Prozessablauf betrieben werden.

**12.** Verfahren nach einem der vorgenannten Ansprüche, wobei die Mindestaufenthaltszeit der Fermentationssubstrate bei der batchweisen anaeroben Methanbildung in jedem Methanfermenter vor der teilweisen Entleerung für wenigstens 10 Tage aufrechterhalten wird.

**13.** Verfahren nach einem der vorgenannten Ansprüche, wobei die anaerobe Methanbildung der zweiten Stufe in wenigstens einem Prozessbehälter mit kreisringförmiger Grundfläche als Nachfermenteranordnung (7) erfolgt und die in der Nachfermenteranordnung (7) der zweiten Stufe der anaeroben Methanbildung vorgesehenen Umwälzeinrichtungen ausschließlich zur intermittierenden Fluidisierung von entstehenden Sedimenten genutzt werden.
